# EUROPEAN PATENT SPECIFICATION

(11) **EP 2 246 077 B1**
(45) Date of publication and mention of the grant of the patent: **28.03.2012**
(21) Application number: 09159182.6
(22) Date of filing: 30.04.2009
(51) Int. Cl.: A61M 1/00

(54) **Medical vacuum aspiration device**
Medizinische Vakuumansaugvorrichtung
Dispositif médical d'aspiration sous vide

(43) Date of publication of application: 03.11.2010
(73) Proprietor: Ipas, Inc., Chapel Hill, North Carolina 27516-4493 (US)
(72) Inventor: Kominski, Terence S., Chapel Hill, NC 27516-4493 (US); Rouleau, Michael P., Chapel Hill, NC 27516-4493 (US)
(74) Representative: Prüfer & Partner GbR European Patent Attorneys

(56) References cited:
- WO-A-81/02523
- WO-A-2008/007892
- DE-A1- 3 005 679
- DE-A1- 19 804 883
- FR-A- 2 627 087
- US-A- 5 667 500
- US-A1- 2007 161 965
- US-B1- 6 264 620

## Description

The invention relates to a medical vacuum aspiration device and a method of detaching a tube from a medical vacuum aspiration device.

It is known to use medical vacuum aspiration devices for removing fluid and tissue from a body cavity. Specifically, medical vacuum aspiration devices are known which can be used several times before they are disposed. Said devices consist of components which can be disassembled.

US 2007 016 1965 discloses a medical vacuum aspiration device consisting of several components which can be easily disassembled and, therefore, easily cleaned and sterilized. Specifically, the medical vacuum aspiration device consists of a collection tube in which the fluid and tissue is collected after a suction process is performed. Additionally, said device has a cannula which is inserted into the body and a plunger used for performing the suction process. The cannula and the collection tube are connected to each other by a fluid conduit inserted into a housing.

The medical vacuum aspiration device mentioned above has the disadvantage that the total medical aspiration device is large. Therefore, it is difficult to transport the fluid and tissue contained in the collection tube in a compact and secure way to other locations for evaluation after the suction process is performed.

WO 2008/007892, US 6,264,620 and DE 3005679 disclose a medical vacuum aspiration device having a tube, a piston which is removably coupled with the displacement means and a closure means, respectively. Said closure means is attachable to the inlet opening of the tube after a suction process is performed by the medical vacuum aspiration device.

The object of the present invention is to provide a medical vacuum aspiration device enabling to transport the suctioned fluid and tissue in a compact and secure way to other locations.

Said object is solved by the features of claim 1 and claim 12, respectively. Further embodiments of the present invention are subject-matter of the dependent claims.

An advantage of the present invention is that the tube can easily be detached from a displacement means because the displacement means is removably coupled with a plunger arranged within the tube. Additionally, an advantage of the invention is that an inlet opening of the tube can be closed by a closure means so that it is secured that no suctioned fluid and tissue can flow out the tube. As a result, the suctioned fluid and tissue can be transported in a secure way. In addition, an advantage of the invention is that the size of the tube is smaller than the size of the total medical vacuum aspiration device so that the suctioned fluid and tissue can be transported in a compact way to another location.

A further advantage of the present invention is that the displacement means can be detached from the piston. Thus, the piston and the displacement means can be easily cleaned, sterilized and reused. In general, a user is free to dispose the medical vacuum aspiration device in total or several components thereof. In the case that the tube and piston are not reused again, a further advantage exists therein that only said components can be disposed. That means that the displacement means can be used several times.

A further advantageous effect can be achieved in that the closure means is arranged at the medical vacuum aspiration device, in particular at the displacement means. With said arrangement it is secured that the closure means does not get lost.

Additionally, an advantage of the present invention is achieved in that the piston provides at least one recess. Said recess is engaged with a protrusion of a stopping element so that it is secured that the piston can not be moved further when the displacement means is removed from the piston.

Details of the invention will be discussed on the basis of the accompanied drawings.
Fig. 1 shows the medical vacuum aspiration device according to the present invention in an exploded view,
Fig. 2 shows a cross sectional view of the closure means,
Fig. 3 shows a cross sectional view of the displacement means,
Fig. 4 shows the piston in a side view,
Fig. 5 shows a cross sectional view of the medical vacuum aspiration device with displacement means being in a retracted position.

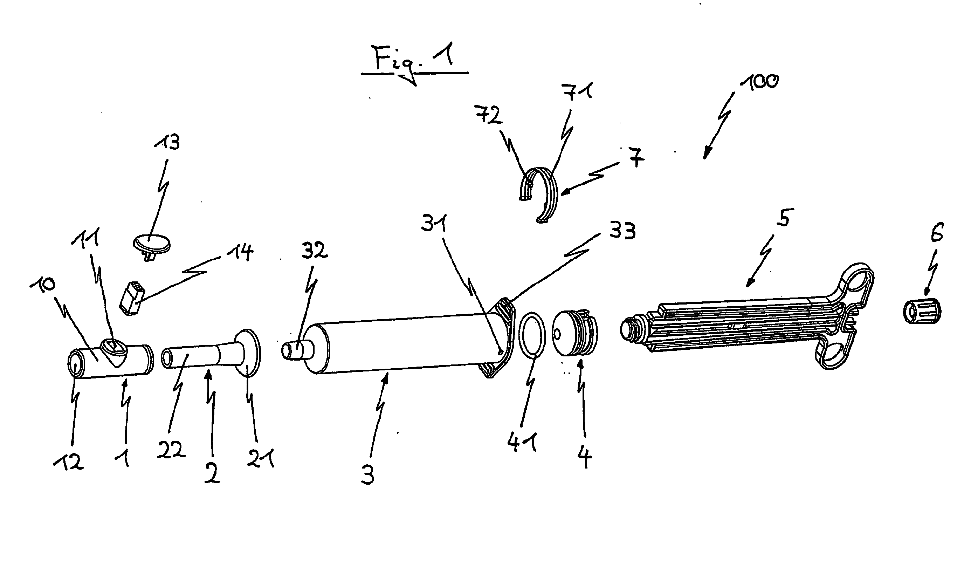

The medical vacuum aspiration device shown in Fig. 1 comprises a displacement means 5, a piston 4 and a closure means 6. The displacement means 5 can be coupled with the piston 4 at one end thereof and can be used to displace the position of the piston 4. The closure means 6 is arranged at the end of the displacement means 5 distal to the piston 4.

Additionally, the medical vacuum aspiration device 100 comprises a tube 3 for collecting the suctioned fluid and tissue. The tube 3 is in the form of a syringe barrel; however, the tube 3 can also have another form than a syringe. In the present embodiment the tube 3 has a cylindrical form and an elongated flange 33 arranged at one end of the tube 3. Said flange 33 can provide a grip for the user during the suction process. At an end of the tube 3, opposite to the elongated flange 33, the tube 3 comprises an inlet opening for enabling the suctioned fluid and tissue to flow into the tube 3. Specifically, the tube 3 comprises a hollow protrusion 32 including said inlet opening. The hollow protrusion 32 extends in a direction away from the tube 3 wherein the suctioned fluid and tissue flows through the hollow protrusion 32 into the tube 3. An inner diameter of the hollow protrusion 32 is smaller than an inner diameter of the tube 3.

At the other end of the tube 3, distal to the hollow protrusion 32, the tube 3 comprises an opening having the same diameter as the tube 3 itself. The piston 4 and the displacement means 5 pass through said opening when they are inserted into the tube 3. In an assembled state of the displacement means 5, the piston 4 and the tube 3, the displacement means 5 is used to displace the piston 4 within the tube 3. The inner diameter of the tube 3 is slightly larger than an outer diameter of the piston 4 so that it is secured that the piston 4 can be moved within the tube 3. The piston 4 comprises a circumferential groove, extending along the circumference of the piston 4, in which a sealing element 41 like e.g. an O-ring is to be arranged. Said sealing element 41 prevents a fluid and tissue flow out of the tube via the piston 4 and does not hinder the movement of the piston 4.

Additionally, the tube 3 comprises at least one hole 31 arranged in its outer surface in the near of the flange 33. The hole 31 is made such that a projection 72, arranged at a stopping element 7 and extending from the stopping element 7, can pass through said hole 31. The stopping element 7 can be connected to the tube 3 at a position proximal to the elongate flange 33 and includes an arcuate body 71 having two free ends and at least one projection 72 located at one of said ends. In the present embodiment, the projection 72 protrudes radially towards the tube 3.

Furthermore, the medical vacuum aspiration device comprises a fluid conduit 2 connected to a cannula which is not shown in Fig. 1. The fluid conduit comprises a first portion 21 and a second portion 22 and is formed of a resilient material. The first portion 21 is arranged at the end of the fluid conduit 2 directed to the tube and radially extends from the second portion 22 such that the first portion 21 has a plate-shaped form. The diameter of the first portion 21 corresponds to the diameter of the tube 3. The second portion 22 of the fluid conduit 2 comprises an inner diameter which is similar to the outer diameter of the protrusion 32 of the tube 3. As a result, the second portion 22 of the fluid conduit can be attached at the protrusion 32 of the tube 3. As the fluid conduit 2 consists of a resilient material the second portion 22 can adjust oneself to the form of the protrusion 32 of the tube 3.

When the fluid conduit 2 is attached at the protrusion 32 of the tube 3, the first portion 21 of the fluid conduit 2 is opposite to the front surface of the tube 3 and encloses said front surface so that the sealing properties between the fluid conduit 2 and the tube 3 improve. As the second portion 22 of the fluid conduit 2 has a similar diameter as the hollow portion of the protrusion 32, the flow of the suctioned fluid and tissue through the fluid conduit 2 into the tube 3 is not hindered by the fluid conduit 2.

A valve element 1 can be attached on the second portion 22 of the fluid conduit 2 in order to control or prevent a flow of the suctioned fluid and tissue into the tube 3 or out of the tube 3. The valve element 1 comprises a pipe element 10 with a hollow 12 having an inner diameter which is marginally larger than an outer diameter of the second portion 22 of the fluid conduit 2 and which is smaller than the diameter of the first portion 21 of the fluid conduit 2. As a result, the valve element 1 can be attached onto the second portion 22 of the fluid conduit 2 so that the valve element 1 is adjacent to the first portion 21 of the fluid conduit 2.

Additionally, the valve element 1 comprises a pressing element comprising a button 13 and a clamp stem 14 wherein the button 13 and the clamp stem 14 can be secured together e.g. by adhesives, press fitting, welding or any other connection means usable to connect said components. The clamp stem 14 is inserted into an opening 11 of the valve element 1 in a direction transversal to fluid flow direction within the valve element 1.

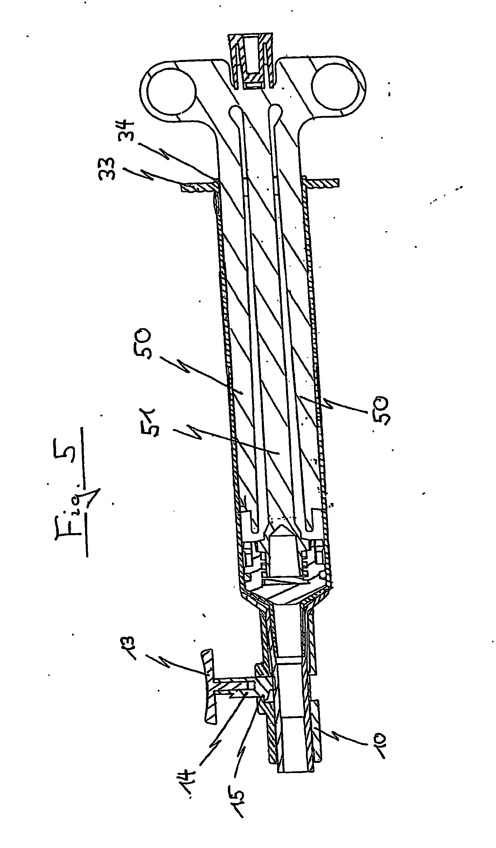

The pipe element 10 comprises a further opening opposite to the opening in which the pressing element is inserted as is evident from Fig. 5. Said further opening enables that, in an actuated state of the pressing element, the fluid conduit 2 can be squeezed within said opening by the clamp stem 14. Thus, the risk of a damage of the fluid conduit 2 due to exerting a press force on the fluid conduit 2 by means of the pressing element is reduced.

As the button 13 is positioned outside the valve element 1 said button 13 can be actuated by a user so that the valve element 1 can be selectively opened and closed. The button 13 can have an oval shape and can include a concave contact surface for engagement by a user's finger to effect opening and closing of the valve element 1. Alternatively, the button can have other shapes.

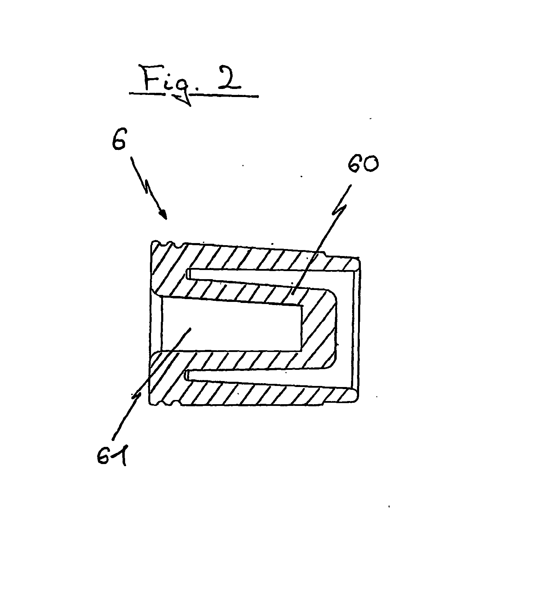

Fig. 2 shows a cross-sectional view of the closure means 6. The closure means 6 comprises a cylindrical form having a hollow. The front surface of the closure means 6 proximal to the displacement means 5 comprises an opening. Additionally, the closure means 6 comprises a protrusion 60 protruding from a front surface distal to the displacement means 5 in a direction towards the front surface proximal to the displacement means 5. The diameter of the protrusion 60 is smaller than an inner diameter of the hollow of the closure means 6 and corresponds to the inner diameter of the hollow of the protrusion 32 of the tube 3. As the diameter of the protrusion 60 of the closure means 6 is equal to the inner diameter of the hollow of the protrusion 32 of the tube 3, it is secured that the fluid and tissue can not flow out the tube when the closure means 6 is attached at the protrusion 32 of the tube 3.

The protrusion 60 protrudes to a position in the near of the front surface of the closure means 6 proximal to the displacement means 5 so that the hollow formed within the closure means 6 has the form of a cup. The protrusion 60 itself comprises a recess 61 extending from the front surface of the closure means 6 distal to the displacement means 5 to a position within the protrusion 60.

An alternative embodiment of the closure means 6 comprises a flange and a protrusion wherein the protrusion extends from the flange. The protrusion is adapted to close the hollow protrusion 32 when the closure means 6 is attached to the tube 3. The diameter of the flange is larger than the diameter of the protrusion 32 so that the flange abuts against the protrusion 32 of the tube when the closure means 6 is attached to said protrusion 32.

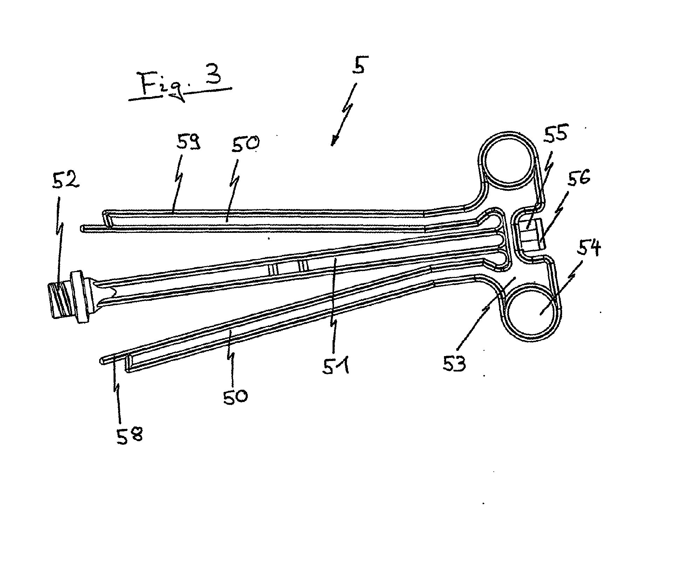

Fig. 3 shows a cross-sectional view of the displacement means 5. The displacement means 5 comprises a handle 53. In the present case the handle 53 comprises two openings 54 . Said openings 54 may be used to enable an easy grasping and moving of the displacement means 5 by the user. Further, a recess is provided at the handle 53 in a rearward middle portion. In said recess a holding element for holding the closure means 6 is provided. In an alternative embodiment, the handle can just be formed as a t-handle or can comprise only one opening.

The holding element 53 comprises at least one projection extending in a direction away from the handle 53. Said projection is suitable to encompass the protrusion 60 of the closure means 6 so that the position of the closure means 6 with regard to the displacement means 5 is fixed when the closure means 6 is attached at the displacement means 5. In the embodiment shown in Fig. 3 two protrusions 55 are provided which can be connected together with a connection portion 56 having the same form as the protrusion 60 when viewed in a direction of a central axis of the displacement means 5.

Furthermore, the displacement means 5 comprises two locking elements 50 and a fixing element 51 extending all from the handle 53 in a direction away from the holding element 53 towards the piston 4. The fixing element 51 is arranged between the locking elements 50 and provides a fixing portion 52 for fixing the displacement means 5 with the piston 4 at its end distal to the handle 53. Specifically, the fixing element 51 comprises a threaded portion for fixing the displacement means 5 in a nut portion of the piston 4. Each of the locking elements 50 provides a first part 58 proximal to the fixing element 51 which is longer than a second part 59 distal to the fixing element 51. The locking elements 50 can expand radially outward when the displacement means 5 is extracted in a position in which the piston 4 abuts against the projections 72 of the stopping element 7 for example (cf. Fig. 1).

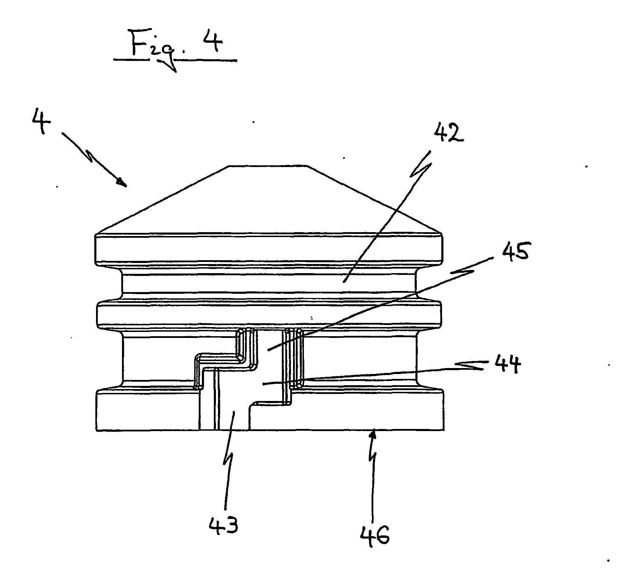

Fig. 4 shows the piston 4 in a side view. The piston 4 comprises a groove 42 in which a sealing ring 41 (cf. Fig. 1) can be inserted. Additionally, the piston 4 comprises at least one recess 43, 44, 45 for securing that the piston 4 can not be moved in a longitudinal direction when the displacement means 5 is detached from the piston 4. The number of the recesses 43, 44, 45 depends on the number of projections 72 of the stopping element 7. The recess comprises a first section 43 parallel with a central axis of the piston 4 and a second section 44 connected with the first section 43 and transversal to said central axis of the piston 4. Additionally, said recess comprises a third section 45 connected with the second section 44 and again parallel with the central axis of the piston 4. Thus, the third section 45 is arranged parallel and offset with regard to the first section 43. Each of said sections 43, 44, 45 is defined by wall portions of the piston 4. The piston 4 also comprises a lower part 46 adapted to abut against the projections 72 of the stopping element 7 when the displacement means 5 is extracted to a specific position.

Fig. 5 shows a cross-sectional view of the medical vacuum aspiration device 100 with the displacement means 5 being in a retracted position. That means that piston 4 is arranged proximal to the valve element 1.

The pressing element of the valve element 1 is not actuated so that a fluid flow is not disturbed by the clamp stem 14 of the pressing element. As can be seen in fig. 5 the locking elements 50 and the fixing element 51 are substantially arranged parallel with each other when the displacement means 5 is in its retracted position. The locking elements 50 and the fixing element 5 stay in said substantial parallel position until the displacement means is extracted to a position in which the second part 59 of the locking element 50 is not guided anymore by the tube.

In said position, each of the locking elements 50 releases radially outward until the first part 58 of the locking elements 50 abuts against an edge 34 (cf. Fig. 5) provided at the end of tube 3 proximal to the flange 33. As a result, it is not possible any longer to push the displacement means 5 towards the tube 3 when the locking elements 50 moved radially outwards because the second part 59 of the locking elements 50 abut against the edge 34 of the tube 3 and, therefore, prevent a further movement of the locking elements 50 and, thus, the displacement means 5. The position of the displacement means 5 in which the locking elements 50 move radially outwards can be the position in which the lower part 46 of the piston 4 abuts against the projections 72 of the stopping element 7. However, it is possible that the locking elements 50 move radially outward in an other position than mentioned-above.

In the following, the operation of the disassembling of the tube 3 from the medical vacuum aspiration device 100 is explained. When the suction process is performed the displacement means 5 and the piston 4 are moved away from the end of the tube 3 proximal to the valve element 1 and the fluid and tissue flows into the tube 3.

In a first step of the disassembling operation, the button 13 of the pressing element is pushed in a direction transverse to the fluid flow and in a direction away from the tube 3 so that the clamp stem 14 of the pressing element 14 is clamped with an edge 15 of the pipe element 10. Thus, no fluid can flow into the tube 3 or out of said tube 3.

In a next step, the valve element 1 and the fluid conduit 2 are disassembled from the tube 3. To achieve this, a force is applied on the valve element 1 in a direction away from the tube 3. When the valve element 1 and the fluid conduit 2 are detached from the tube 3 the closure means 6 is detached from the holding element of the displacement means 5 and attached to the protrusion 32 of the tube 3. As a result, said closure means 6 prevents that the fluid and tissue can flow out from the protrusion 32 of the tube 3.

In a next step, the stopping element 7 is attached to the tube so that the projections 72 pass through the holes 31 of the tube 3. Then the displacement means 5 is extracted until the lower part 46 of the piston 4 abuts against the projections 72 of the stopping element 7.

Afterwards, the displacement means 5 is rotated until the projections 72 of the stopping element 7 are engaged with the first section 43 of the recess of the piston 4. Then the displacement means 5 is again extracted in the direction away from the tube 3 until the projections 72 of the stopping element 7 abut against a wall portion of the first section 43 of the piston 4. Thereafter, the displacement means 5 are rotated until the projections 72 of the stopping element abut against a wall portion of the second section 44 of the piston 4. Then, the displacement means 5 are again pulled in a direction away from the tube 3 until the projections 72 of the stopping element 7 abut against a wall portion of the third section 45.

As a result, the displacement means 5 is in a position where it can be detached. Additionally, it is secured that the piston 4 can not be moved in a longitudinal direction anymore because in such case the projections 72 of the stopping element 7 would abut against one of the wall portion of the recess second and/or third section 44, 45 of the piston 4.

In a last step, the displacement means 5 is rotated against the threading direction so that the displacement means 5 is uncoupled from the piston 4.

## Claims

1. Medical vacuum aspiration device (100) comprising a tube (3), a movable piston (4) and a displacement means (5)
wherein the piston (4) is arranged within the tube (3),
wherein the tube (3) comprises an inlet opening at one end side thereof,
the displacement means (5) is removably coupled with the piston (4) and in that a closure means (6) is attachable to the inlet opening of the tube (3) after the suction process is performed by the medical vacuum aspiration device (100)
**characterized in that**
the piston (4) comprises at least one recess (43, 44, 45) which is engaged with a protrusion of a stopping element (7) when the displacement means (5) is removed from the piston in order to prevent a rotation of the piston (4) and a further movement thereof.

2. Medical vacuum aspiration device (100) according to claim 1 **characterized in that** the closure means (6) is detachably attached to the medical vacuum aspiration device (100) before the suction process is performed by the device (100).

3. Medical vacuum aspiration device (100) according to claim 1 or 2 **characterized in that** the closure means (6) is detachably attached to an end of the displacement means (5) distal to the piston (4).

4. Medical vacuum aspiration device (100) according to claim 3 **characterized in that** the end of the displacement means (5) has at least one holding element for holding the closure means (6), said holding element is protruding from said end to a direction away from the piston (4).

5. Medical vacuum aspiration device (100) according to one of the preceding claims **characterized in that** the closure means (6) has a protrusion adapted to close the inlet opening of the tube (3) and a flange wherein the protrusion extends from the flange.

6. Medical vacuum aspiration device (100) according to one of the preceding claims **characterized in that** the diameter of the inlet opening of the tube (3) is smaller that the diameter of the tube.

7. Medical vacuum aspiration device (100) according to one of the preceding claims **characterized in that** the recess comprises a first section (43) being parallel to a central axis of the piston (4), a second section (44) being transverse to said central axis and connected with the first section (43) and a third section (45) being parallel with the central axis and offset with regard to the first section (43) and connected with the second section (44).

8. Medical vacuum aspiration device (100) according to one of the preceding claims **characterized in that** a protrusion (32) is provided at the end of the tube (3) including the inlet opening.

9. Medical vacuum aspiration device (100) according to claim 8, **characterized in that** a fluid conduit (2) is attached on the protrusion (32) of the tube (3).

10. Medical vacuum aspiration device (100) according to claim 9 **characterized in that** the fluid conduit (2) comprises a first portion (21) and a second portion (22) wherein a diameter of the second portion (22) is larger than a diameter of the first portion (21).

11. Medical vacuum aspiration device (100) according to claim 9 or 10 **characterized in that** a valve element (1) is detachably provided on the fluid conduit (2).

12. Method of disassembling a tube (3) from a medical vacuum aspiration device (100) comprising the following steps:
- Detaching of components (1, 2) arranged at the end of the tube (3) distal to a displacement means (5),
- Attaching a closure means (6) to an opening of the tube (3)
- Detaching the displacement means (5) from the piston (4), **characterized in that**
a stopping element (7) is arranged at the tube (3) so that a protrusion of the stopping element (7) is engaged with at least one recess (43, 44, 45) of the piston (4) before the displacement means (5) is detached from the piston (4) in order to fix the piston (4) and to prevent a rotation of the piston (4).

13. Method of disassembling a tube (3) from a medical vacuum aspiration device (100) according to claim 12 wherein the displacement means (5) is extracted to a position in which the piston (4) abuts against projections (72) of a stopping element (7) before the displacement means (5) is rotated.

## Patentansprüche

1. Medizinische Unterdruckansaugvorrichtung (100), welche eine Röhre (3), einen bewegbaren Kolben (4) und ein Verschiebungsmittel (5) aufweist,
wobei der Kolben (4) innerhalb der Röhre (3) angeordnet ist,
wobei die Röhre (3) eine Einlassöffnung an einem ihrer Enden aufweist,
das Verschiebungsmittel (5) lösbar mit dem Kolben (4) verbunden ist, und dass ein Verschlussmittel (6) an die Einlassöffnung der Röhre (3) angebracht werden kann, nachdem der Ansaugprozess durch die medizinische Unterdruckansaugvorrichtung (100) durchgeführt wurde,
**dadurch gekennzeichnet, dass**
der Kolben (4) zumindest eine Vertiefung (43, 44, 45) aufweist, welche mit einem Vorsprung eines Stopperelements (7) ineinander greift, wenn das Verschiebungsmittel (5) von dem Kolben gelöst wird, um einer Rotation des Kolbens (4) und einer weiteren Bewegung dessen vorzubeugen.

2. Medizinische Unterdruckansaugvorrichtung (100) nach Anspruch 1, **dadurch gekennzeichnet, dass** das Verschlussmittel (6) lösbar an die medizinische Unterdruckansaugvorrichtung (100) angebracht wird, bevor der Ansaugprozess durch die Vorrichtung (100) durchgeführt wird.

3. Medizinische Unterdruckansaugvorrichtung (100) nach Anspruch 1 oder 2, **dadurch gekennzeichnet, dass** das Verschlussmittel (6) lösbar an ein Ende des Verschiebungsmittels (5) distal von dem Kolben (4) angebracht ist.

4. Medizinische Unterdruckansaugvorrichtung (100) nach Anspruch 3, **dadurch gekennzeichnet, dass** das Ende des Verschiebungsmittels (5) zumindest ein Halteelement zum Halten des Verschlussmittels (6) hat, wobei das Halteelement von dem Ende in eine weg weisende Richtung von dem Kolben (4) vorsteht.

5. Medizinische Unterdruckansaugvorrichtung (100) nach einem der vorhergehenden Ansprüche, **dadurch gekennzeichnet, dass** das Verschlussmittel (6) einen Vorsprung hat, der dazu angepasst ist, die Einlassöffnung der Röhre (3) zu verschließen, und einen Flansch, wobei der Vorsprung sich von dem Flansch erstreckt.

6. Medizinische Unterdruckansaugvorrichtung (100) nach einem der vorhergehenden Ansprüche, **dadurch gekennzeichnet, dass** der Durchmesser der Einlassöffnung der Röhre (3) kleiner ist, als der Durchmesser der Röhre.

7. Medizinische Unterdruckansaugvorrichtung (100) nach einem der vorhergehenden Ansprüche, **dadurch gekennzeichnet, dass** die Vertiefung einen ersten Abschnitt (43) aufweist, welcher parallel zu einer Mittelachse des Kolbens (4) ist, einen zweiten Abschnitt (44), welcher quer zu der Mittelachse und mit dem ersten Abschnitt (43) verbunden ist, und einen dritten Abschnitt (45), welcher parallel zu der Mittelachse und in Bezug auf den ersten Abschnitt (43) versetzt und mit dem zweiten Abschnitt (44) verbunden ist.

8. Medizinische Unterdruckansaugvorrichtung (100) nach einem der vorhergehenden Ansprüche, **dadurch gekennzeichnet, dass** an dem Ende der Röhre (3) ein Vorsprung (32) vorgesehen ist, der die Einlassöffnung enthält.

9. Medizinische Unterdruckansaugvorrichtung (100) nach Anspruch 8, **dadurch gekennzeichnet, dass** eine Fluidleitung (2) an dem Vorsprung (32) der Röhre (3) angebracht ist.

10. Medizinische Unterdruckansaugvorrichtung (100) nach Anspruch 9, **dadurch gekennzeichnet, dass** die Fluidleitung (2) einen ersten Bereich (21) und einen zweiten Bereich (22) aufweist, wobei ein Durchmesser des zweiten Bereichs (22) größer als ein Durchmesser des ersten Bereichs (21) ist.

11. Medizinische Unterdruckansaugvorrichtung (100) nach Anspruch 9 oder 10, **dadurch gekennzeichnet, dass** ein Ventilelement (1) lösbar an der Fluidleitung (2) vorgesehen ist.

12. Verfahren zum Zerlegen einer Röhre (3) einer medizinischen Unterdruckansaugvorrichtung (100), welche die folgenden Schritte aufweist:
- Lösen der Komponenten (1, 2), welche an dem Ende der Röhre (3) distal von dem Verschiebungsmittel (5) angeordnet sind,
- Anbringen eines Verschlussmittels (6) an eine Öffnung der Röhre (3)
- Lösen des Verschlussmittels (5) von dem Kolben (4), **dadurch gekennzeichnet, dass**
ein Stopperelement (7) an der Röhre (3) angeordnet ist, so dass ein Vorsprung des Stopperelements (7) mit zumindest einer Vertiefung (43, 44, 45) des Kolbens (4) ineinander greift, bevor das Verschlussmittel (5) von dem Kolben (4) gelöst wird, um den Kolben (4) zu befestigen und einer Rotation des Kolbens (4) vorzubeugen.

13. Verfahren zum Zerlegen einer Röhre (3) einer medizinischen Unterdruckansaugvorrichtung (100) nach Anspruch 12, wobei das Verschiebungsmittel (5) in eine Stellung, in welcher der Kolben (4) gegen Vorsprünge (72) eines Stopperelements (7) stößt, herausgezogen wird, bevor das Verschiebungsmittel (5) gedreht wird.

## Revendications

1. Dispositif médical d'aspiration sous vide (100) comprenant un tube (3), un piston mobile (4) et des moyens de déplacement (5),
dans lequel le piston (4) est agencé à l'intérieur du tube (3), dans lequel le tube (3) comprend une ouverture d'entrée au niveau de son côté d'extrémité,
les moyens de déplacement (5) sont couplés de manière amovible avec le piston (4) et en ce que les moyens de fermeture (6) peuvent être fixés à l'ouverture d'entrée du tube (3) après que le processus d'aspiration a été réalisé par le dispositif d'aspiration de vide médical (100),
**caractérisé en ce que** :
le piston (4) comprend au moins un évidement (43, 44, 45) qui est mis en prise avec une saillie d'un élément de butée (7) lorsque les moyens de déplacement (5) sont retirés du piston afin d'empêcher une rotation du piston (4) et un autre mouvement de ce dernier.

2. Dispositif médical d'aspiration sous vide (100) selon la revendication 1, **caractérisé en ce que** les moyens de fermeture (6) sont fixés de manière détachable au dispositif médical d'aspiration sous vide (100) avant que le processus d'aspiration ne soit réalisé par le dispositif (100).

3. Dispositif médical d'aspiration sous vide (100) selon la revendication 1 ou 2, **caractérisé en ce que** les moyens de fermeture (6) sont fixés de manière détachable à une extrémité des moyens de déplacement (5) à distance du piston (4).

4. Dispositif médical d'aspiration sous vide (100) selon la revendication 3, **caractérisé en ce que** l'extrémité des moyens de déplacement (5) a au moins un élément de support pour supporter les moyens de fermeture (6), ledit élément de support fait saillie de ladite extrémité dans une direction à distance du piston (4).

5. Dispositif médical d'aspiration sous vide (100) selon l'une quelconque des revendications précédentes, **caractérisé en ce que** les moyens de fermeture (6) ont une saillie adaptée pour fermer l'ouverture d'entrée du tube (3) et un rebord, dans lequel la saillie s'étend à partir du rebord.

6. Dispositif médical d'aspiration sous vide (100) selon l'une quelconque des revendications précédentes, **caractérisé en ce que** le diamètre de l'ouverture d'entrée du tube (3) est plus petit que le diamètre du tube.

7. Dispositif médical d'aspiration sous vide (100) selon l'une quelconque des revendications précédentes, **caractérisé en ce que** l'évidement comprend une première section (43) qui est parallèle à un axe central du piston (4), une deuxième section (44) qui est transversale par rapport audit axe central et raccordée avec la première section (43) et une troisième section (45) qui est parallèle à l'axe central et décalée par rapport à la première section (43) et raccordée à la deuxième section (44).

8. Dispositif médical d'aspiration sous vide (100) selon l'une quelconque des revendications précédentes, **caractérisé en ce qu'**une saillie (32) est prévue à l'extrémité du tube (3) comprenant l'ouverture d'entrée.

9. Dispositif médical d'aspiration sous vide (100) selon la revendication 8, **caractérisé en qu'**un conduit de fluide (2) est fixé sur la saillie (32) du tube (3).

10. Dispositif médical d'aspiration sous vide (100) selon la revendication 9, **caractérisé en ce que** le conduit de fluide (2) comprend une première partie (21) et une deuxième partie (22), dans lequel un diamètre de la deuxième partie (22) est plus grand qu'un diamètre de la première partie (21).

11. Dispositif médical d'aspiration sous vide (100) selon la revendication 9 ou 10, **caractérisé en ce qu'**un élément de soupape (1) est prévu de manière détachable sur le conduit de fluide (2).

12. Procédé pour démonter un tube (3) d'un dispositif médical d'aspiration sous vide (100) comprenant les étapes suivantes consistant à :
- détacher les composants (1, 2) agencés à l'extrémité du tube (3) à distance des moyens de déplacement (5),
- fixer des moyens de fermeture (6) à une ouverture du tube (3),
- détacher les moyens de déplacement (5) du piston (4),
**caractérisé en ce que** :
- un élément de butée (7) est agencé au niveau du tube (3) de sorte qu'une saillie de l'élément de butée (7) est agencée avec au moins un évidement (43, 44, 45) du piston (4) avant que les moyens de déplacement (5) ne soient détachés du piston (4) afin de fixer le piston (4) et d'empêcher une rotation du piston (4).

13. Procédé pour démonter un tube (3) d'un dispositif médical d'aspiration sous vide (100) selon la revendication 12, dans lequel les moyens de déplacement (5) sont extraits dans une position dans laquelle le piston (4) vient en butée contre les saillies (72) d'un élément de butée (7) avant que les moyens de déplacement (5) ne soient entraînés en rotation.
